# EUROPEAN PATENT SPECIFICATION

(11) **EP 3 960 234 B1**
(45) Date of publication and mention of the grant of the patent: **30.10.2024**
(21) Application number: 20193781.0
(22) Date of filing: 01.09.2020
(51) Int. Cl.: A61N 5/06

(54) **IRRADIATION THERAPY DEVICE FOR SELF-TREATING PARKINSON'S DISEASE**
THERAPIEGERÄT ZUR PARKINSON SELBST-BEHANDLUNG
THÉRAPIE PAR IRRADIATION D'AUTO-TRAITEMENT DE LA MALADIE DE PARKINSON

(43) Date of publication of application: 02.03.2022
(73) Proprietor: nanomaX, 9500 Villach (AT)
(72) Inventor: HALLER, RUDOLF, 9500 Villach (AT); HUANG, Jiajin, 523 Taipei (TW); CHEN, Hsi-Nan, 523 Taipei (TW)

(56) References cited:
- TW-B- I 702 067
- US-A1- 2011 092 863
- US-A1- 2018 008 839
- US-A1- 2019 070 431

## Description

### Background

Parkinson's disease (PD) is an asymmetric neurodegenerative disease affecting the dopaminergic neurons especially in the basal ganglia causing cardinal motor symptoms as bradykinesia, tremor, and rigidity, leading to a progressive loss of nerve cells in the brain especially at substantia nigra. Those affected suffer from speech disorders, mood swings, reduced mobility and the muscle tremor.

First line treatment for PD is generally by drug therapy using dopamine replacement medications. Once the right dose for each person is established the medication generally is controlling the symptoms of Parkinson's. Over time however; the effects of drugs often are no longer sufficient to manage the symptoms. Side effects can develop along with disease progression. PD treatment traditionally has been to manage symptoms, not to modify the disease by slowing or stopping it or to create any neuro-protections. Patients diagnosed since 10 years with PD consume on average a dose of 700 mg till up over 1000 mg max. of dopamineregic drugs, with the danger of a severe side effect, known as "freezing", where the patient is suddenly in a paralysis, unable to move forward, which makes it desirable to keep the necessary medication as low as possible.

Although Parkinson's disease is known since more than 200 years, no cure is available, but several approaches of treatment are on market and under evaluation, where invasive and non-invasive methods are applied.

Invasive methods mainly are DBS (deep brain stimulation ) and stem cell therapies, non-invasive therapies mostly use sound or magnetic and electric waves to stimulate brain cells. Examples of common non-invasive therapies are electro-magnetic-based Transcranial Magnetic Stimulation (TMS), in which the nerve cells in the brain are activated or even inhibited via magnetic fields, or Transcranial Puls Stimulation ( TPS ) applying light pulses on a limited skull area to stimulate nerve cells, or Focused Ultra Sonic (FUS) therapy, mechanically interrupting nerve channels responsible for uncontrolled hand tremor by overheating a focused brain area to destroy specific nerve cells.

A specific non-invasive therapy for wound-healing, skin-treatment, pain relieve, hair-grow is using semiconductor emitters like Light Emitting Diodes (LED) or Vertical Cavity Surface Emitting Laser (VCSEL), mostly with visible and near infra-red light spectrum, and named as Low-Light-Level-Theraypy (LLLT), Photo-Bio-Modulation Therapy ( PBMT ) and other termini, covering all the same approach: influencing cell beneficial behavior by light.

The influence of red or near infrared- as well as blue light on mammalian cells is observed as supporting element for the production of Adenosine Tri Phosphate (ATP), which is commonly referred to as the "power house" or "energy currency" of the cell, as it provides energy, and breakdown of ATP through hydrolysis, serves a broad range of functions in the cell, including signaling and DNA/RNA synthesis, where the influence of absorbance of photogenic energy on the signaling pathway of exosomes is under evaluation, further also leading to a regulation of Reactive Oxygen Species ( ROS ), Nitric Oxide ( NO ) and Inter cellular Calcium ( ICa2+ ).

Boosting additional NO generation light energy of a bandwidth of 405 nm ±20 nm is known and analyzed in several studies.

Light energy is absorbed by chromophores inside the mitochondria, supporting the Cytochrome C Oxidase ( CCO ) at complex V of the Electron Transport Chain ( ETC ) of the mitochondrion inner membrane converting Adenosine Duo Phosphate ( ADP ) to ATP at the ATP synthase , but not being exclusively primary receptor for near infrared light.

### PRIOR ART STUDIES and PUBLICATIONS

Available animal or human studies are describing beneficial outcome of different irradiation-therapies with light-sources in wavelength range from red- infra-red- and blue light bandwidth, without disclosure of a useful portable device to treat Parkinson patients.
C.L. Hamilton, J Mitrofanis e.a. Australia " light -bucket"
C. Hamilton, D.Hamilton, e.a.
M.R. Hamblin , Fahrzad Salehpoure.a GER, USA "PD animal study
S. Rutten, Vrije University, NETHERLAND

Furthermore, document US2011/0092863 A1 provides a helmet-shaped device configured to irradiate patient's scalp using light in four different wavelength ranges.

### PRIOR ART DEVICES

On the market are available -Photo-Bio-Modulation-devices, for home-use with portable conception, or stand-alone-design, are based on semi-spherical helmet shaped devices containing single mounted light sources ( LEDs or laser in numbers of at about 250 pieces max.), mostly claiming beneficial hair growth effects, as well as " supporting brain health ", or hand-held therapy-devices with only few irradiation LEDs or lasers, which are placed by a therapist on the head of patients. Even therapy devices constructed by hobbyists, like a bucket containing simple red light LED-stripes are known to be used by patients, and described as " light bucket ".

A FDA approved Clinical Trial ( NCT02175472 )Photo Pharmics Spectramax USA; is using a table-top lamp emitting green/blue light, with outcomes, even questioned by the researcher. Although several animal and human studies showed beneficiary effects on brain injuries as well on Parkinson's disease, current devices are without conception to penetrate skull and brain tissue in a sufficient stimulating way with different power-settings, radiant energy, tight placements of light-sources, and optimized head coverage, together with sequential flashing modes, which is base of underlying invention.

### Field of invention

Medical treatment of Parkinson's Disease ( PD ) by trans-cranial irradiation.

The invention and its most advantageous embodiments are defined in the appended set of claims.

The technical problem, solved by underlying invention is to apply irradiation on human head to penetrate hair, scalp, skull and brain tissue in a sufficient depth to ignite biochemical processes, well described in plural studies in vitro as in vivo. Considering optimal power, power-modification, irradiation placement as well as irradiation placement modification, irradiation-wavelength, together with environmental conditions hygienically factors, and wear-ability of the device is the core of disclosed invention.

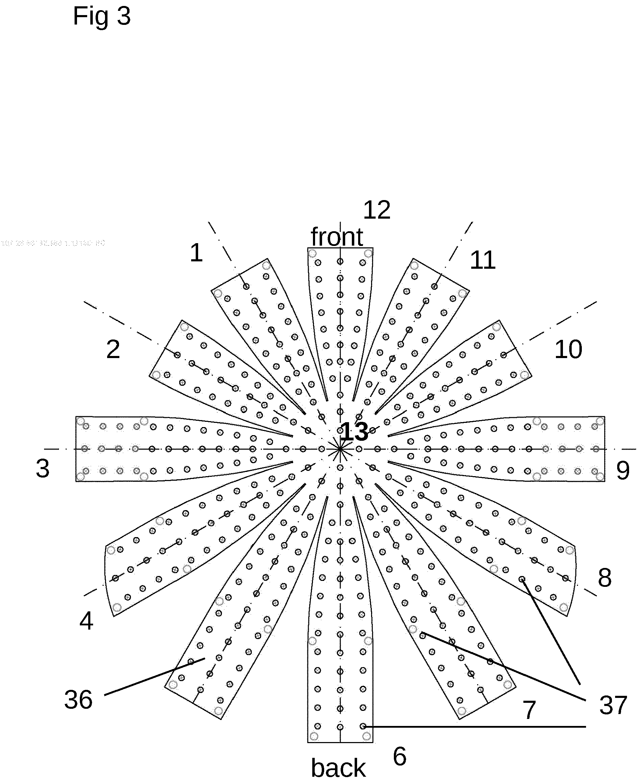

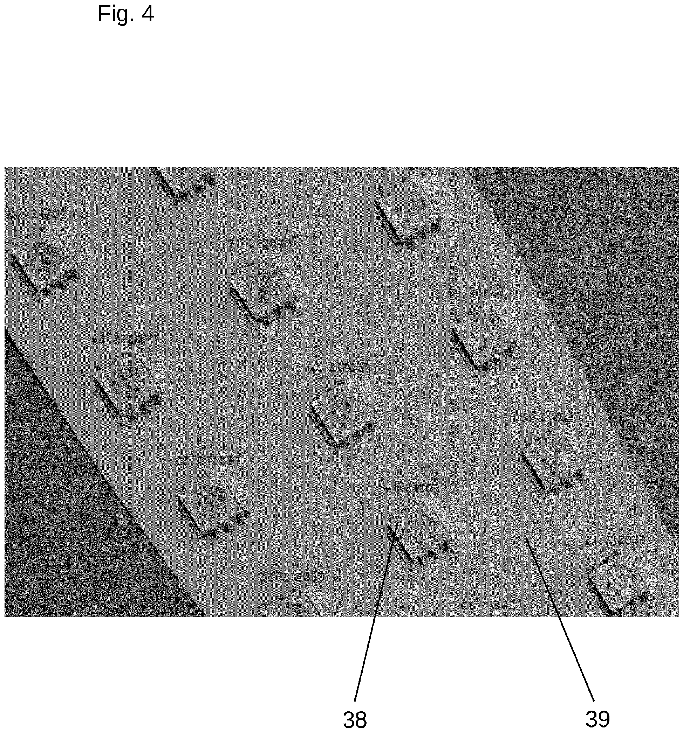

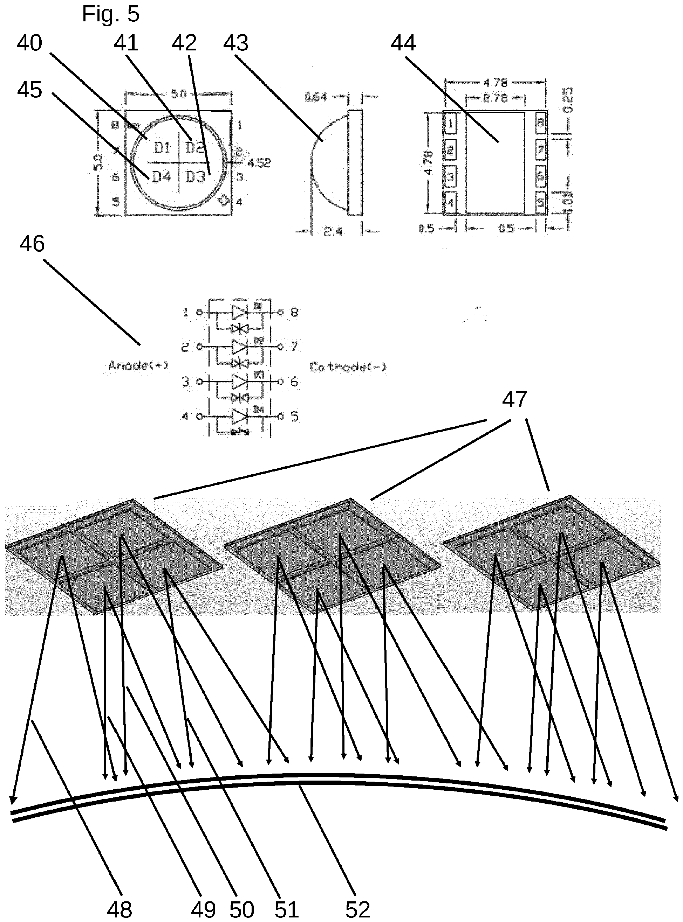

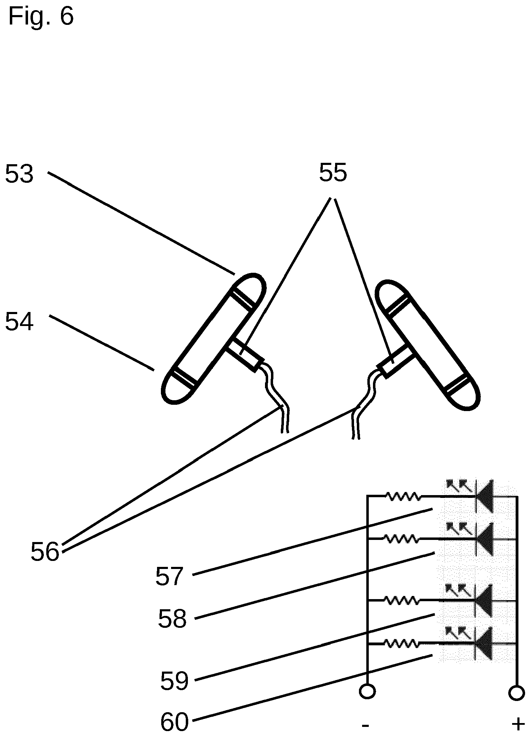

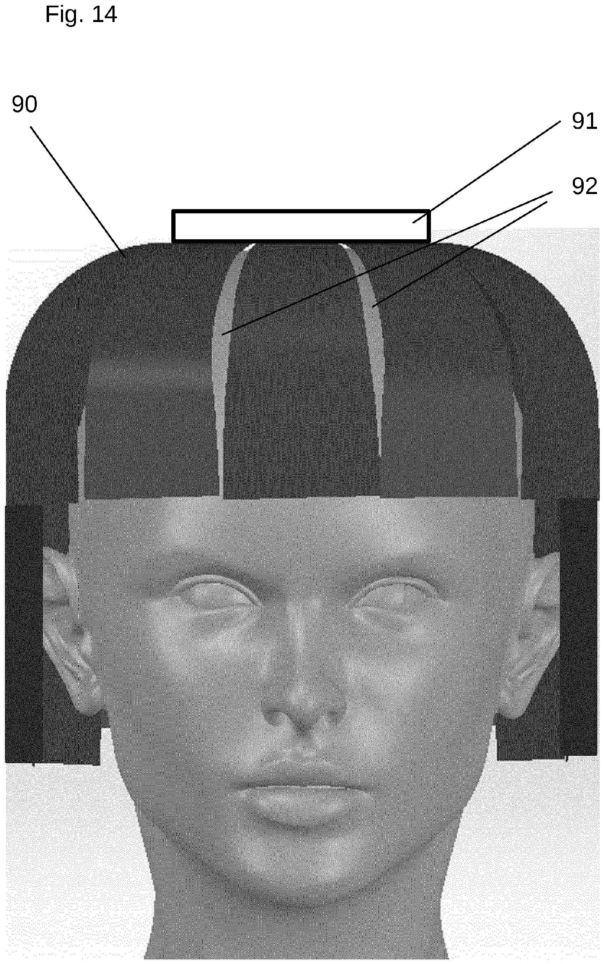

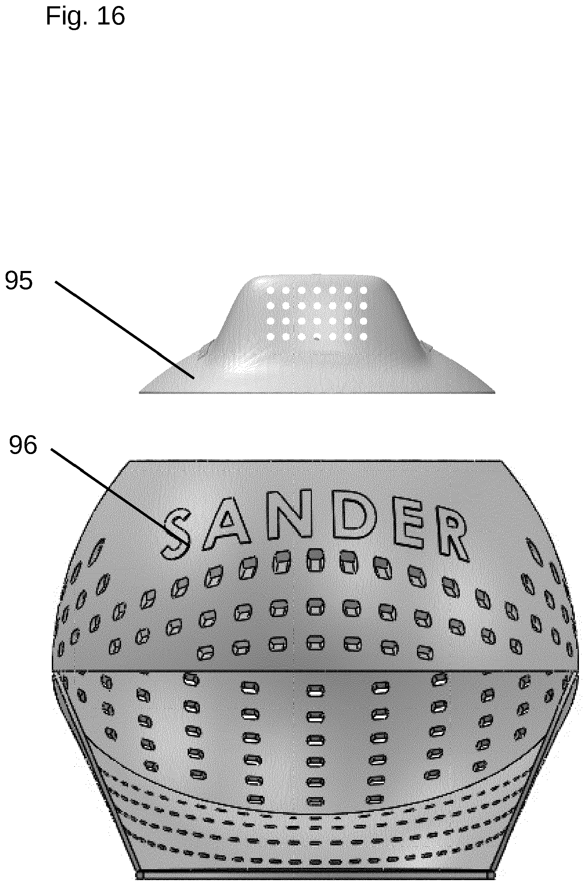

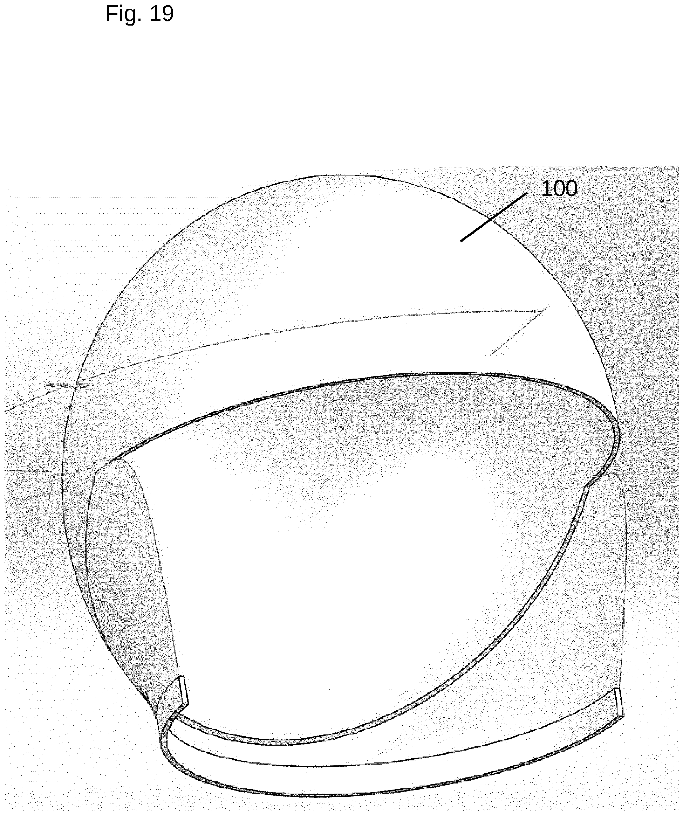

The invention is comprising a portable-, wearable helmet-shaped head cover Fig. 16, containing an outer shell base-ABS part 96 , a removable outer-shell top-ABS part 95 , with a combined Light Emitting Diode ( LED ) array Fig. 3 and an integrated LASER module Fig. 6 , as illustrated in Fig. 14 a flexible printed circuit board (PCB ) 90 , shaped according a spherical cutting pattern to achieve sufficient ventilation slots 92 , with an onboard Master Control Unit ( MCU ) 91 placed under the removable outer shell top ABS part, as well as sketched in Fig 3 includes 1440 Light Emitting Diodes ( LED ) as non-coherent light sources in bandwidth of 660 nm ±20 nm 40, 850 nm ±20 nm 41, 1550 nm ±20 nm 42 and 405 nm ±20 nm 45 shown in Fig. 5, with drawing of side-view 43 and bottom-view 44 together with electrical chart of the quadruple light source 46 , mounted as single chip on a 5050 LED socket 47 thereafter soldered on a PCB as surface mounted devices (SMD) as shown in Fig. 4. An optical grade transparent Polymethyl Methacrylate ( PMMA ) inner shell, Fig. 19 , protecting the emitting light sources, assuring irradiation without loss and hygienic clean ability for multiple usage.

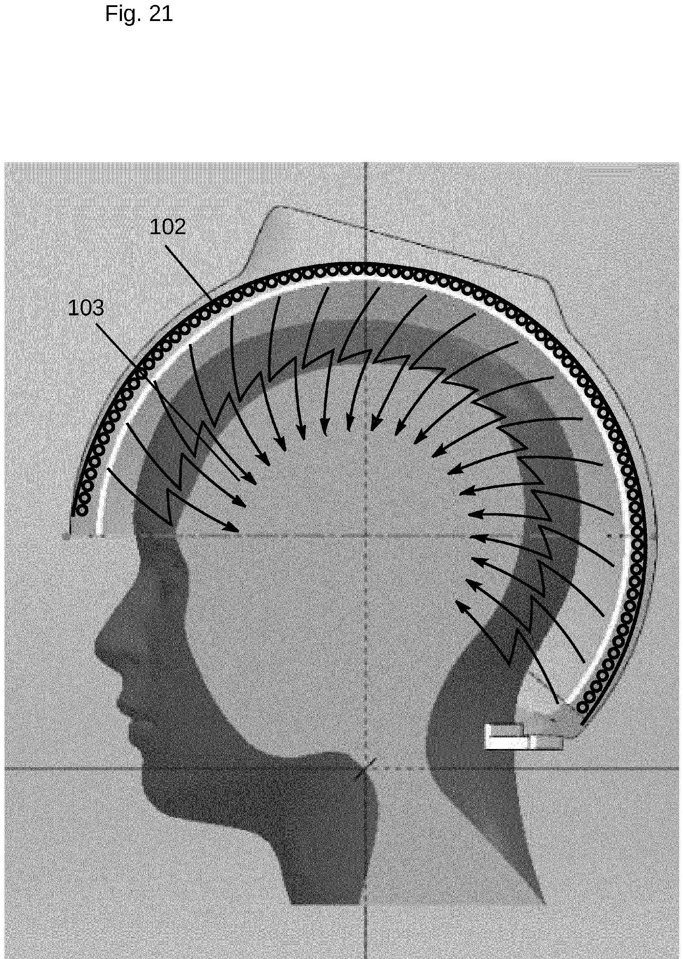

The device is intended to support sub-cellular neuronal bio-chemical processes leading in reduction of stiffness, movement disorders, and promoting relaxation of muscles, whereas the VCSEL laser adapter at ear delivers normal radiation therapy through the skull, but on nose is intended to breach the blood-brain-barrier( BBB ) allowing drugs delivery via the nasal cavity inside the brain, known as Intranasal Nose-to Brain Delivery. The full size helmet style, shown in Fig. 21, is covering forehead, ears, and back head till to neck, with a full irradiation influence on total skull 103 emitted bv tight arranged light sources 102.

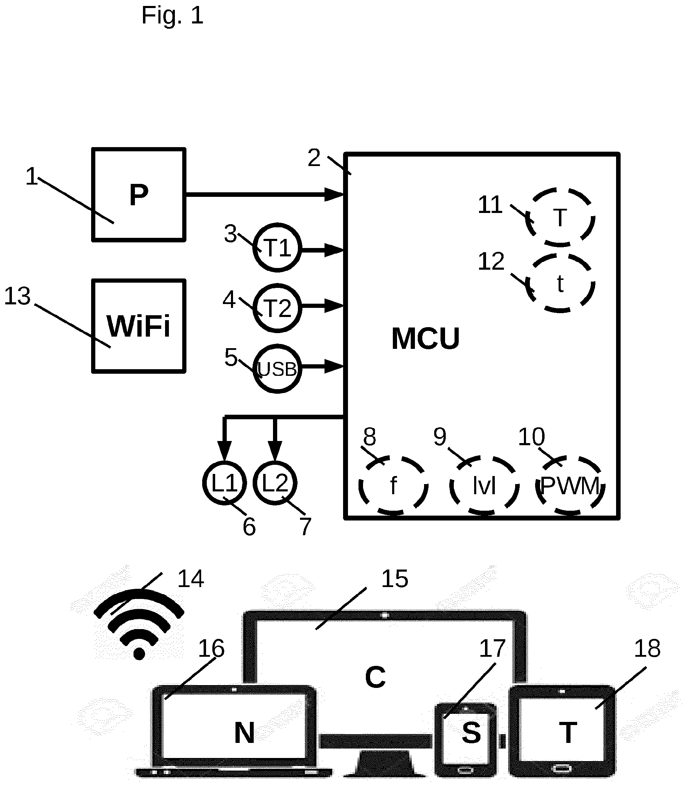

Fig. 1 shows the electronic schema of the underlying patent comprising a 12V/20 Ah powerbank 1 , an 32 bit Advanced RISC Machine ( ARM ) Micro Controller Unit ( MCU ) 2 , Touch buttons for power- 3 and mode- 4 -input, an Unified Serial Bus ( USB ) 5 for software update, laser module output for laser modules 6 ,7 , the software controlled frequency rhythm 8, power level 9, by Pulse Wide Modulation .10 , a Temperature security shutdown regulator 11, a Timer module 12, a Wi-Fi router 13, communicating through Wi-Fi 14 , with controlling devices as PC 15, or Notebook 16, or Smartphone 17 , and Tablet 18 , operating on Windows, iOS, Linux, and Android software.

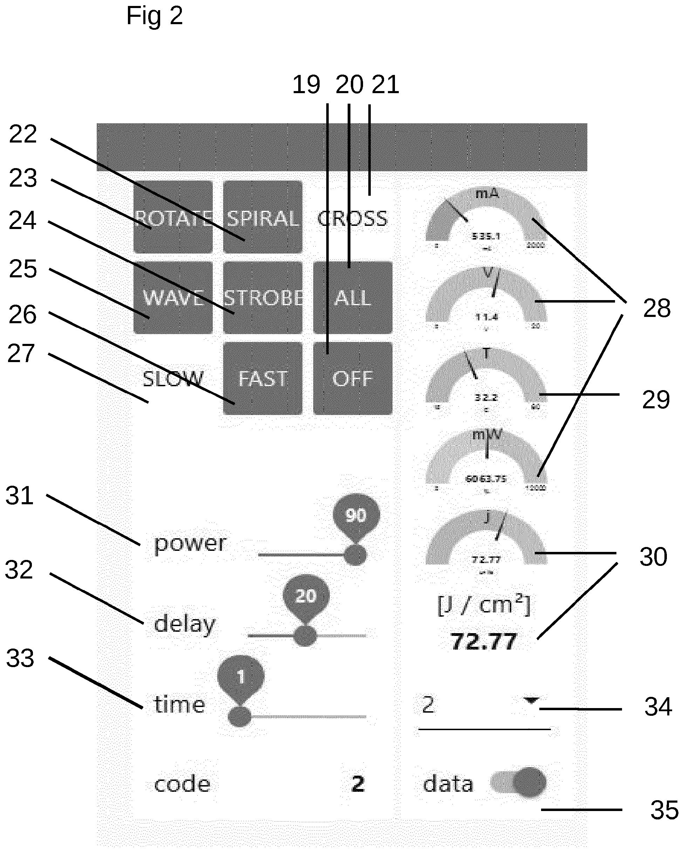

Fig. 2 shows the Graphical User Interface ( GUI ) used to control parameters of the invention like ON / OFF switch 19 , full flashing mode, blinking all 1440 pieces LEDs 20 , a flashing mode with diagonal- 21, spiral- 22 , rotation 23, strobe- 24, wave- 25 irradiation-pattern and rhythm, as well a preset frequency rhythm as fast 26 or slow 27 . With the slider "power" 31 irradiation power is adjusted, with slider " delay" 32 , delay time between increment steps of power is selected, with slider " time " 33 therapy time is chosen. Electrical values are displayed on scales 28 , inside helmet temperature is indicated at scale 29 , real time irradiation energy shown on scale 30 , transmission channel of actual controlled device ( in simultaneous multi-device / multi-channel operation mode ) is selected by drop-down menu 34 , and data recording is activated by switch 35.

Fig. 3 is a drawing of the flexible PCB inner side 36 , which is faced to skull, ( flat, unfolded ) with 360 pieces of SMD mounted LED sockets comprising quadruple light sources of totaling 1440 individual light sources.

Fig. 4 is a magnified presentation of the flexible PCB inner side ( flat, unfolded ) 39 , showing LED socket 38 mounted as LED array on a single segment..

Fig. 5 is a top view of the four light sources 40 , 41 , 42, and 45, with a side view 43, and bottom view 44 of the LED socket, together with their electric symbolic chart 46 . Also demonstrating irradiation beams emitted by the quadruple light sources, mounted on a single LED socket 47 , illuminating the skull surface 52 with symbolized irradiation beam of 660 nm ±20 nm 48 , 850 nm ±20 nm 49 ,1550 nm ±20 nm 50 ,and 450 nm ±20 nm 51 .

Fig. 6 shows the two ear/ nose laser modules 55, comprising the laser itself 53 with Infra-Red wavelength of 850 nm ±1 nm, together with green indication LED 54 , and the electrical symbol chart of laser 1 57, laser function-indicator 1 58, laser 2 59 and laser function-indicator 2 60 , allowing simultaneously the usage of head-, and ear- or nose- irradiation .

Fig. 7 is showing the outer-, or top-side-flexible PCB, facing away from head, containing 5 pieces of frontal segments 61, 7 pieces of side and back segments 62, and 1 center-top segment 63 .

Fig. 8 shows mounted MCU 64 and driver Integrated Circuits ( IC ) 65 of center-top-segment..

Fig. 9 shows a symbolic electrical chart, illustrating " irradiation segment 1" 66, " irradiation segment 2 " 67 , "irradiation segment 12 " 68 , and " irradiation segment 13 ", as well as laser 1 70, indicator 1 71, laser 2 72, indicator 2 73 .

Fig. 10 illustrates the incremental process during power-rise 74 , the overlaying 40 Hz pulse frequency 75 , and the 0.25 Hz pulsing frequency of laser modules 76.

Fig. 11 shows characteristic wavelength data of LED 660 nm ±20 nm 77, of LED 850 nm ±20 nm 78, of LED 1550 nm ±20 nm 79 and of LED 405 nm ±20 nm 80 .

Fig. 12 shows characteristic wavelength data of laser 850 nm ±1 nm 81, the illumination angle thereof 82 , and the typical illumination angle of implemented LEDs. 83 .

Fig. 13 depict the different illumination modes controlled by MCU," rotate "84 , incrementing circular 4 segments in cycle, " spiral " 85 , incrementing circular segments in cycle," wave " 86 , incrementing alternating circular 4 segments in cycle," cross " 87 , incrementing diagonal segments in cycle," strobe " 88 , incrementing randomized segments in cycle," full" 89 , pulsing all segments in cycle,

Fig. 14 shows the bent flexible PCB covering full forehead, till back to neck, including ears 90 , and top mounted MCU 91 , constructed to allow ventilation channels 92 for cooling.

Fig. 15 shows the combined wearable helmet cover without the between inner- 94 and outer shield 93, mounted flexible PCB.

Fig. 16 shows the upper- 95 , and lower-part 96 , of the outer shell of the helmet, which covers the flexible PCB on top.

Fig. 17 shows the left side of the device with touch button for flash mode selection 98 and plug for laser module 97.

Fig. 18 is the back view with the USB plug 99 used for software upload.

Fig. 19 is the drawing of the inner shell 100 , protecting the flexible PCB, manufactured in clear optical PMMA to reduce optical losses to a minimum.

Fig. 20 shows, in cross section view, the tight assembly of LED light sources 101 covering the head from forehead till back to neck, including ears.

Fig. 21 symbolizes, in cross section view, the deep penetration of light energy till into the brain tissue 103 , emitted by light sources 102 .

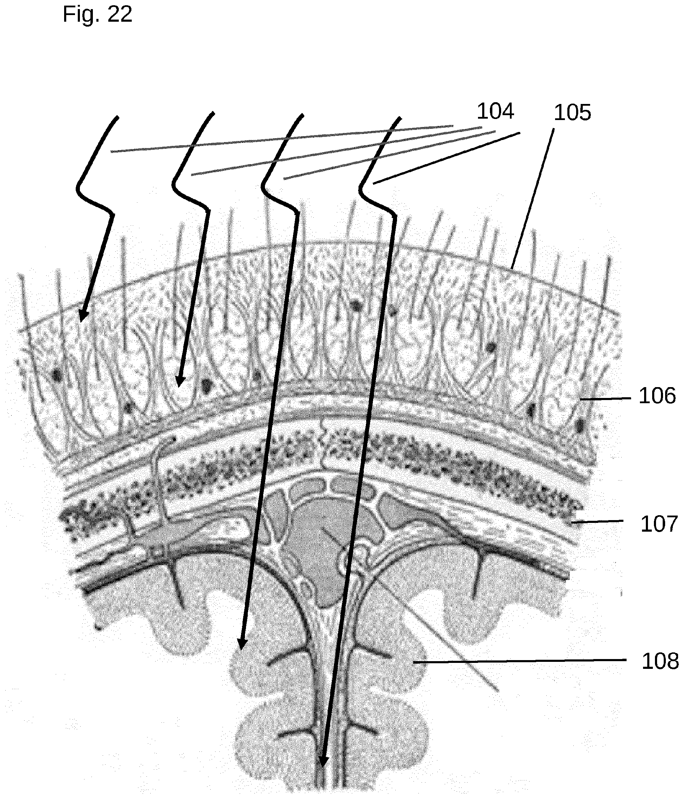

Fig. 22 demonstrates four different light beams 104 , with different wavelength, penetrating between hairs 105 , through scalp 106, skull 107 and brain tissue 108 in different depths, where higher wavelength assures deeper penetration, but blue light activates surface of scalp only. To achieve full penetration in different brain areas 4 different wavelength chips, namely 660 nm ±20 nm, 850 nm ±20 nm, 1550 nm ±20 nm and 405 nm ±20 nm are integrated on a single LED socket, delivering high intensity therapeutic 40 Hz pulsed photon energy, with a maximum irradiation flux intensity of 64 mW/cm², 154 J/cm² ( Root Mean Square (RMS)), on a full treatment surface of 188 cm² .

Fig. 23 shows the bio-chemical reaction caused by the light irradiation in different wavelengths, resulting for example in a positive stimulation of Adenosine Tri Phosphate ( ATP ) production in phase V 110 of the Electron Transport Chain ( ETC ) 109 on the inner membrane of mitochondria ( 111 ), but also stimulating for example Reactive Oxygen Species ( ROS ) or Nitric Oxide ( NO ).

Fig. 24 is a topographic chart of the brain activities, where lighter color areas 112 correspondent to lower, and darker colored areas 113 to higher brain activities, measured at 19 electrodes, placed according the electrode schemes 114 .

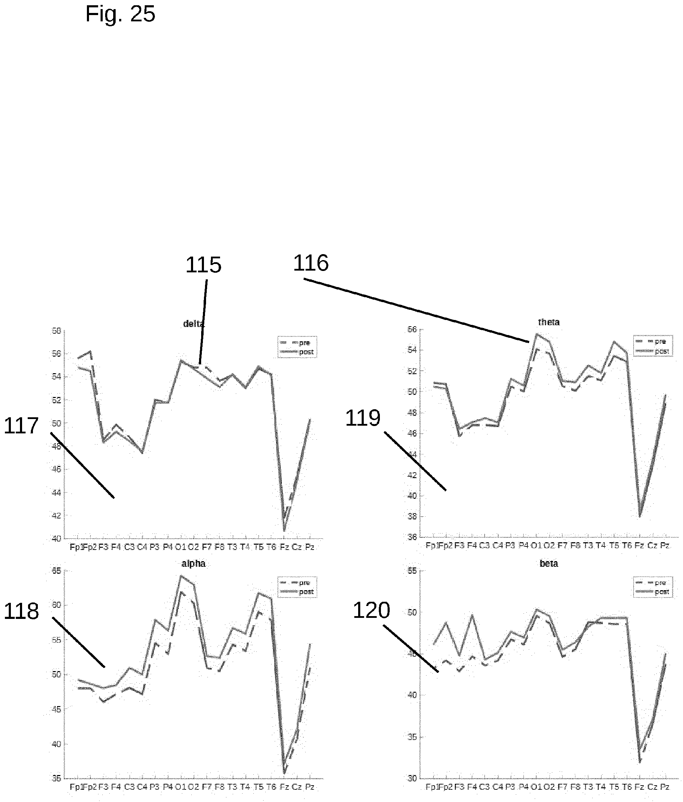

Fig. 25 is showing the result of an Electroencephalogram ( EEG ) analysis, which was recorded before, and after 20 min irradiation, plotting brainwaves in Alpha bandwidth ( 9 - 14 Hz) 118, Beta bandwidth ( 15 - 30 Hz ) 120 , Delta Bandwidth ( 1 - 3 Hz ) 117 , and Theta bandwidth ( 4- 8 Hz ) 119 charted before " pre " 115 and after " post" 116 irradiation, illustrating a significant increase of Alpha activities, signaling calm stable relaxation without of signs of anxiousness, an increase of Beta activities, especially on forehead demonstrating an awake activity, and slight increase in Theta activity, prescribed as a status of "deep relaxation ", demonstrating positive influence of underlying invention on brain activities after irradiation.
Fig. 1 is a schematic overview of electronic components of an irradiation device related to the embodiment.
Fig. 2 depicts a screenshot of the Graphical User Interface ( GUI ) of the device's remote-controller interface.
Fig. 3 is a top view of the flexible PCB, inner side ( unshaped, in flat position ), showing the placement of light sources of the radiation device.
Fig. 4 is a magnified image of arranged Light Emitting Diodes ( LEDs).
Fig. 5 is a drawing with top-, side- and bottom view of a single light source module, and electrical chart thereof, additional sketch, symbolizing the overlapping irradiation coverage of scalp.
Fig. 6 is a sketch depicting two ear / nose adapter laser modules, as also their electrical chart.

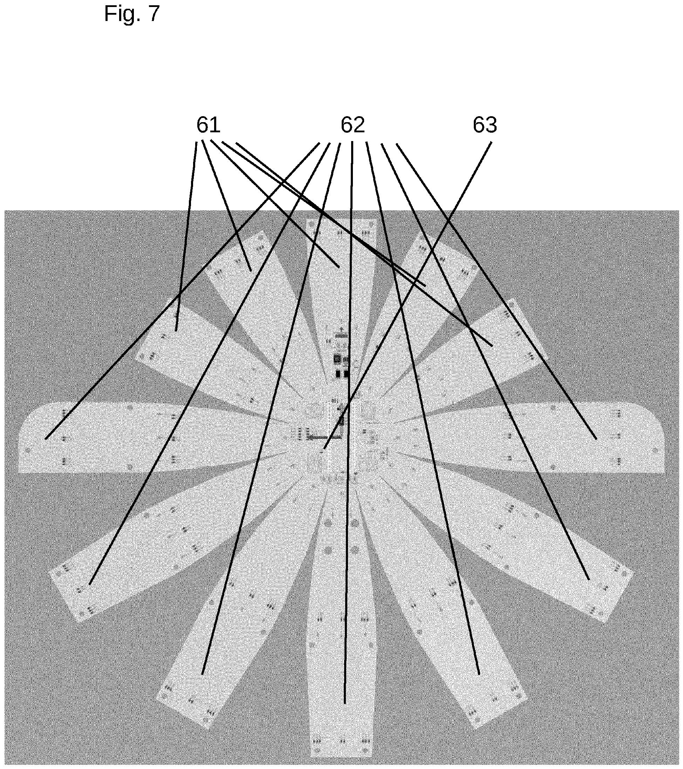

Fig. 7 is showing the flexible PCB, complete upper side ( unshaped, in flat position ), with electronic control units and drivers mounted on top.

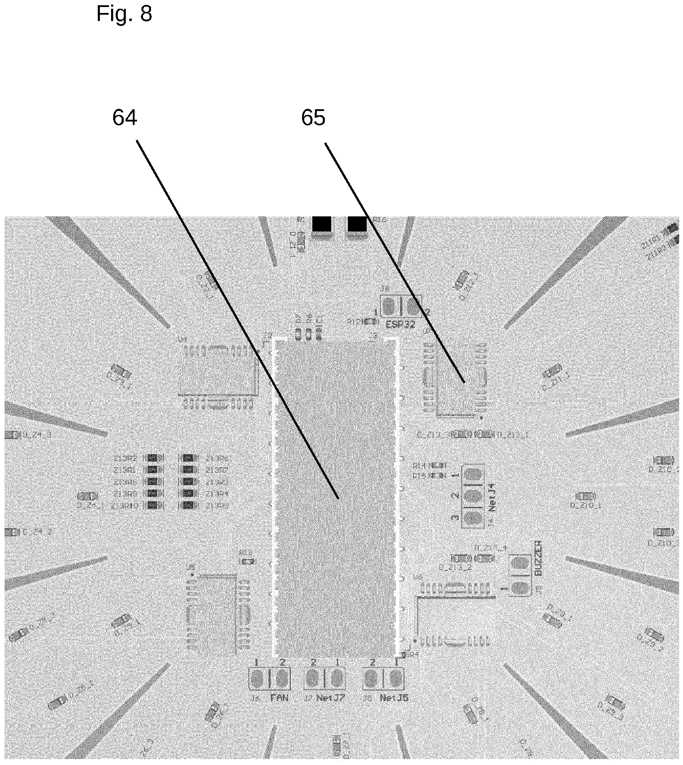

Fig. 8 is showing the flexible PCB, center segment, upper side ( unshaped, in flat position ), with MCU and drivers mounted on top.

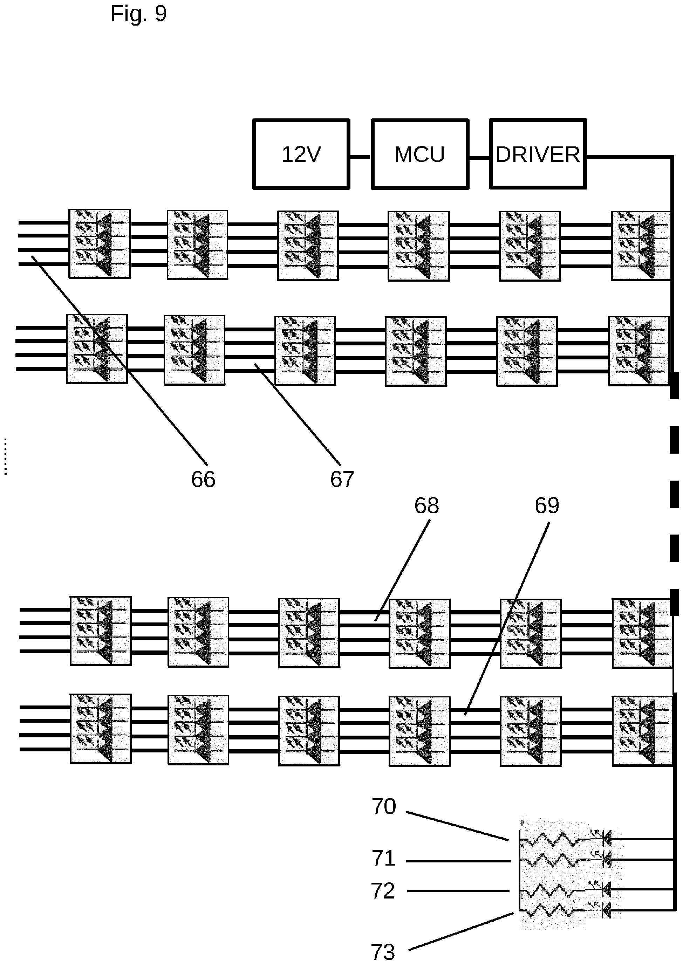

Fig. 9 symbolizes the independent controlled 13 irradiation segments, as well as the ear/laser-module.

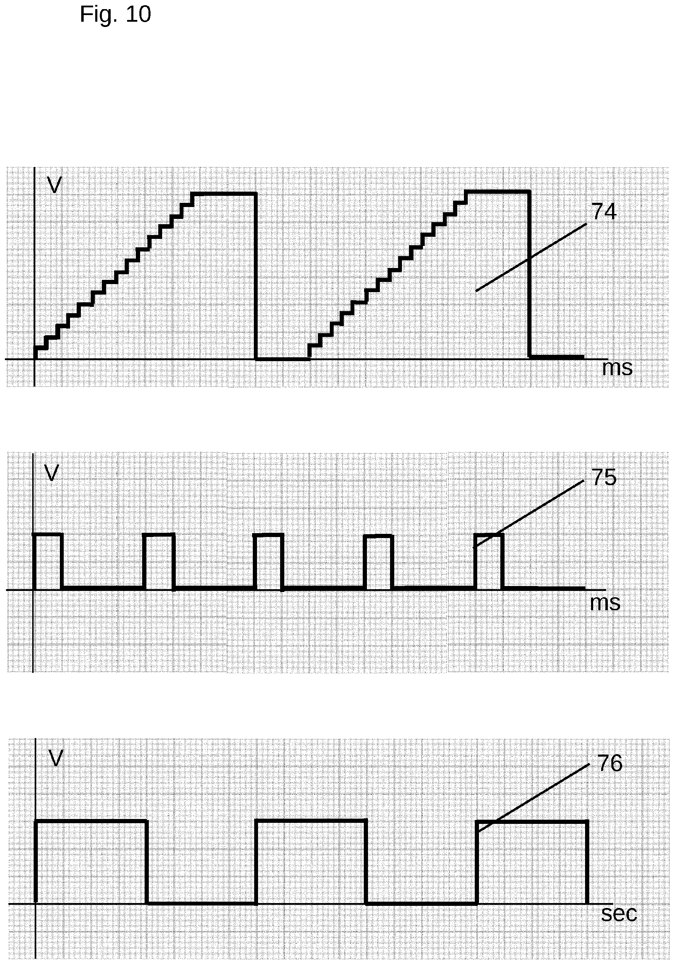

Fig. 10 illustrates electrical signal properties demonstrating power increase and pulsing.

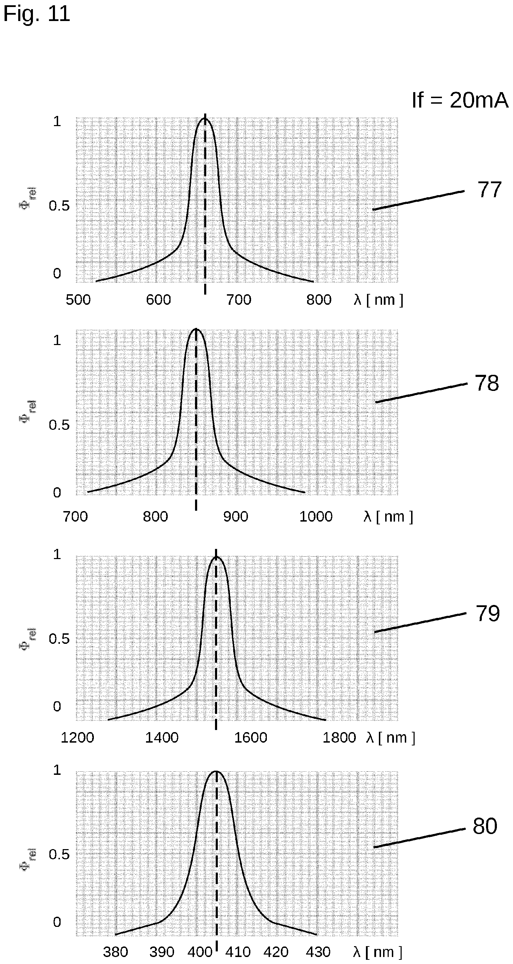

Fig. 11 are irradiation characteristics of implemented LEDs .

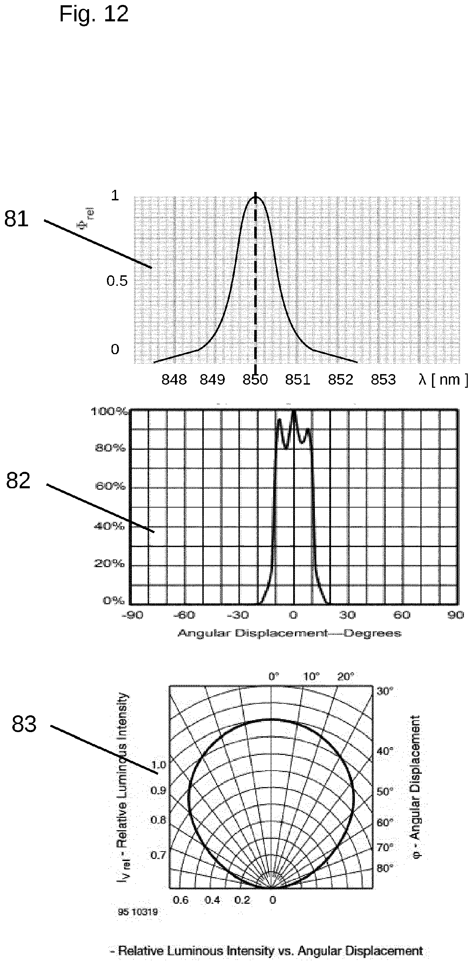

Fig. 12 are characteristics of ear / nose laser module light source, and LED irradiation angle data.

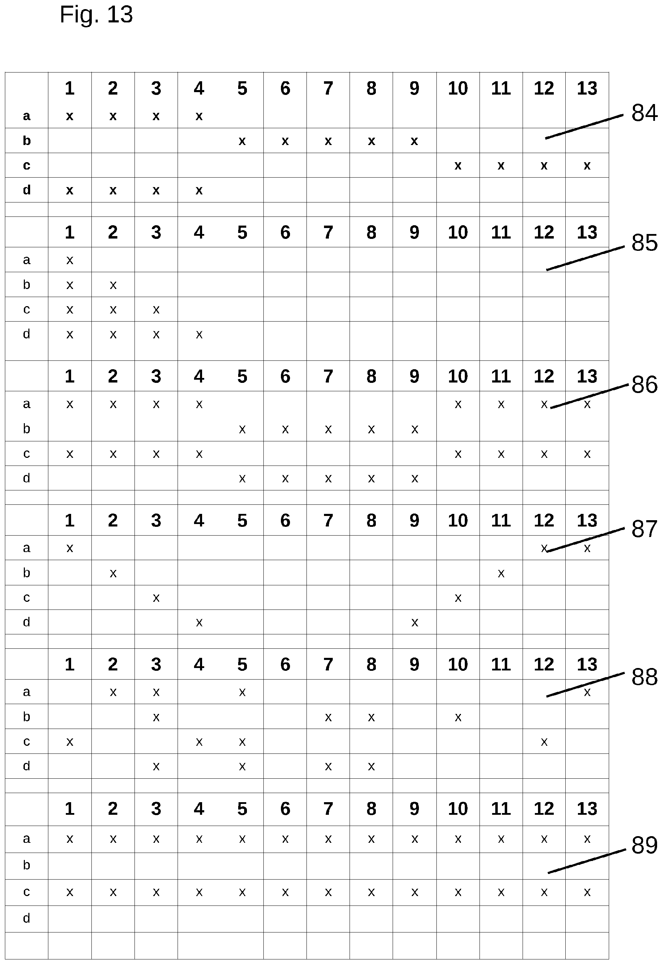

Fig. 13 shows various flashing modes of the irradiation device.
Fig. 14 illustrates the bending of the flexible PCB segments to cover head.

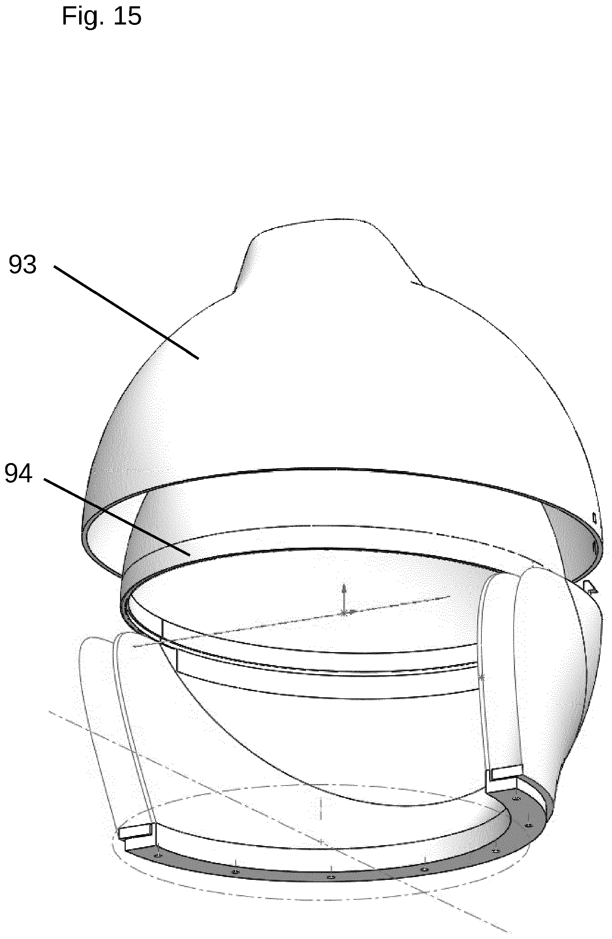

Fig. 15.. is a 3D view of inner and outer helmet parts.
Fig. 16 is the front view of outer helmet parts ( with detached top part).

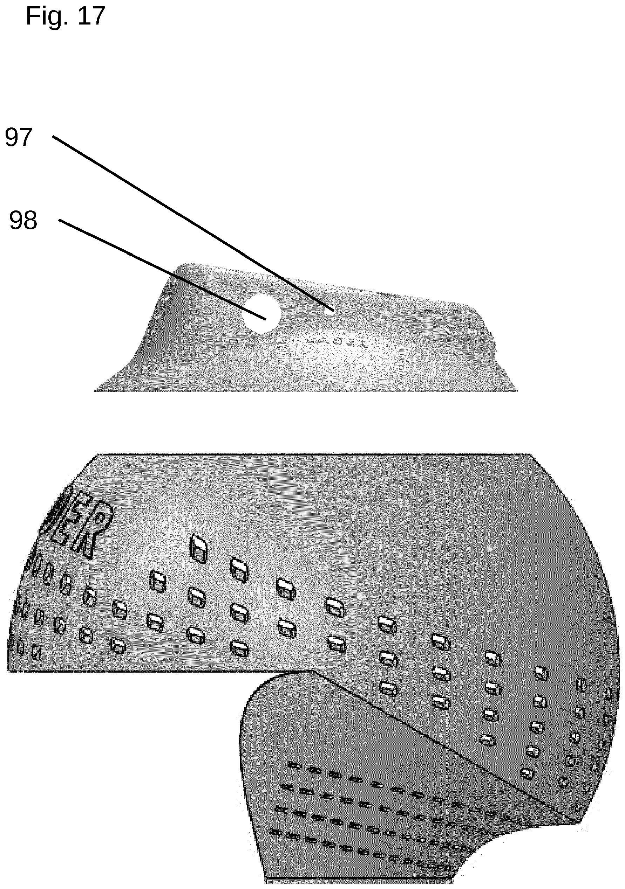

Fig. 17 is the side view of outer helmet parts showing arrangement of touch-button and laser plug ( with detached top part ).

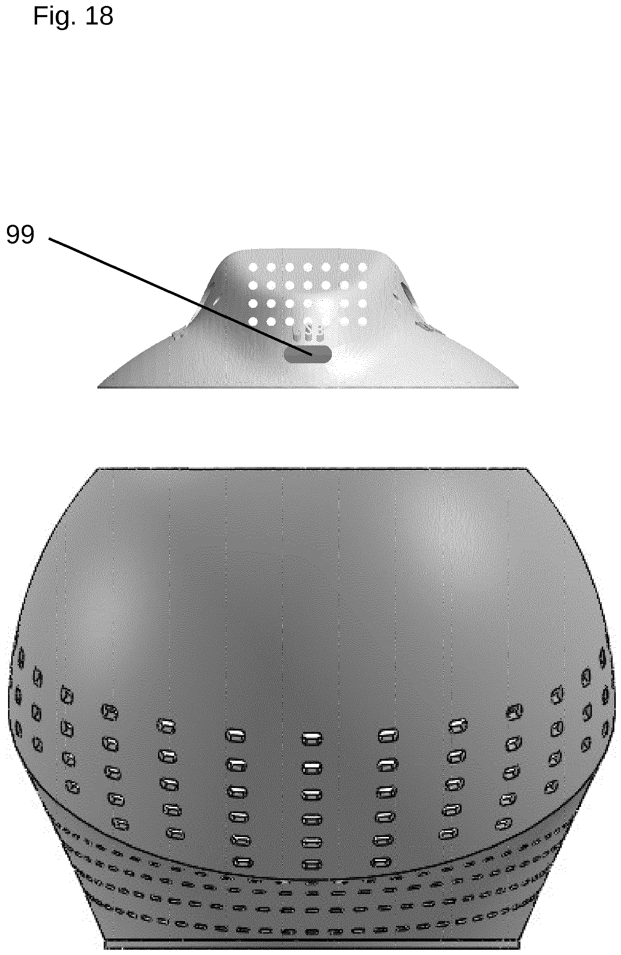

Fig. 18 is the back view of outer helmet parts showing USB plug ( with detached top part ).
Fig. 19 is a 3 D-view of the inner optical transparent PMMA helmet part.

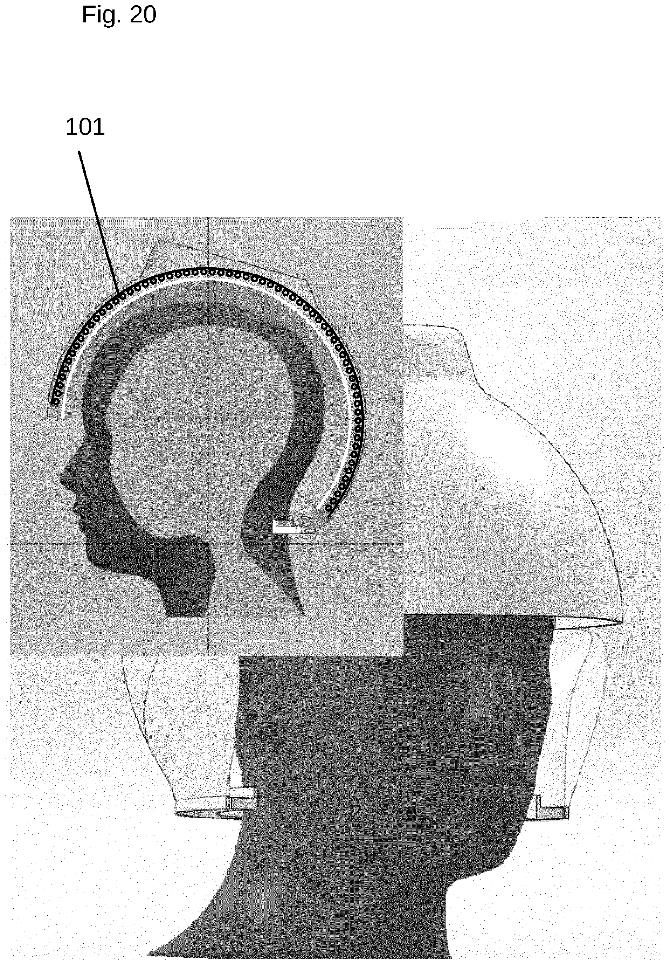

Fig. 20 shows a cross section and a 3D-view of the helmet device placed on a head.
Fig. 21 is a cross section of the irradiation helmet illustrating the full coverage of head, and placement of irradiation sources, as there penetration through skull.
Fig. 22 is a cross section of head, with parts of brain symbolizing photogenic penetration through hair, scalp, skull inside the brain.

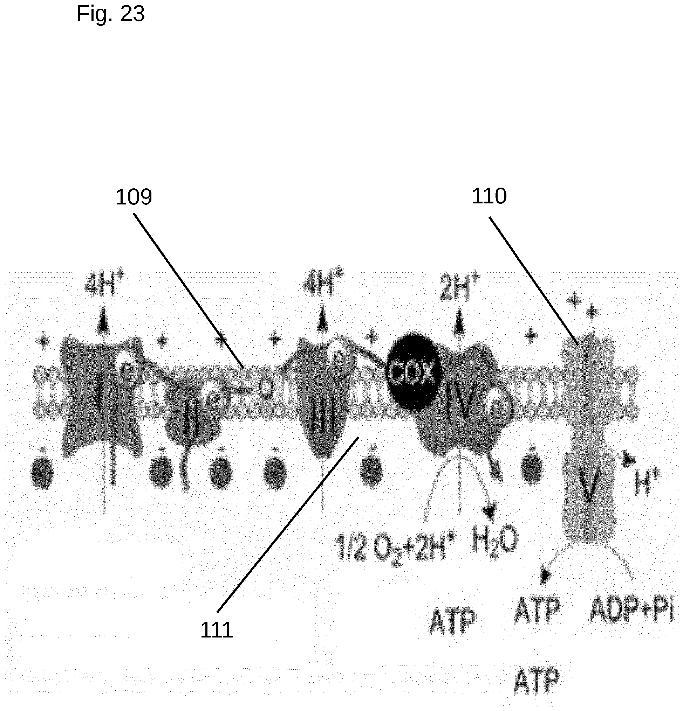

Fig. 23 is a drawing of the inner membrane of mitochondria, focused on the electric transport chain ( ETC ) .

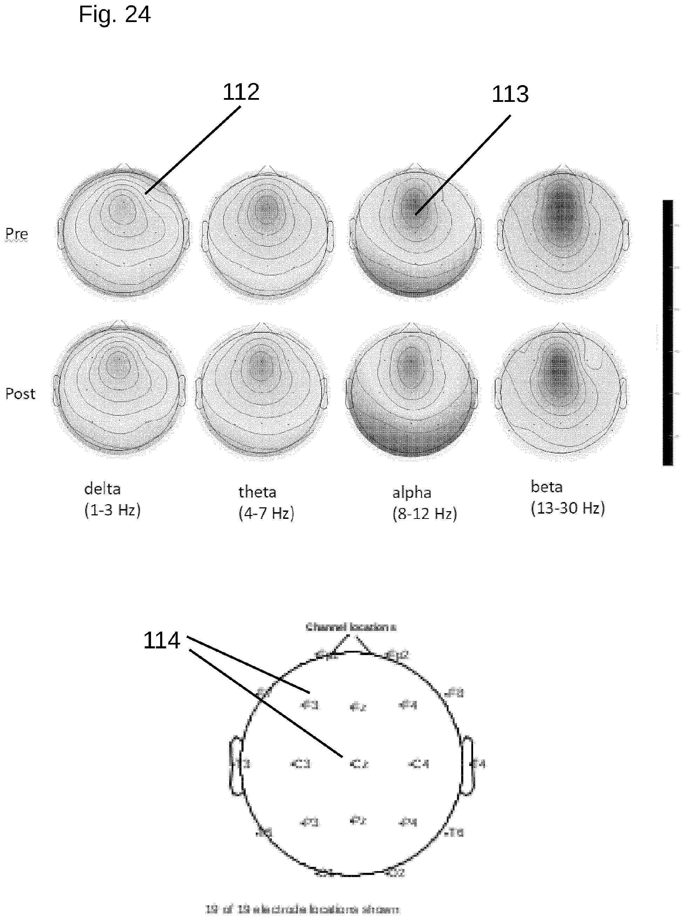

Fig. 24 shows pre and post irradiation results of Electroencephalogram ( EEG ) separated by different brainwaves as a topographical EEG scalp map.
.Fig. 25 shows pre and post irradiation results of Electroencephalogram ( EEG ) separated by different brainwaves as single electrode charts.

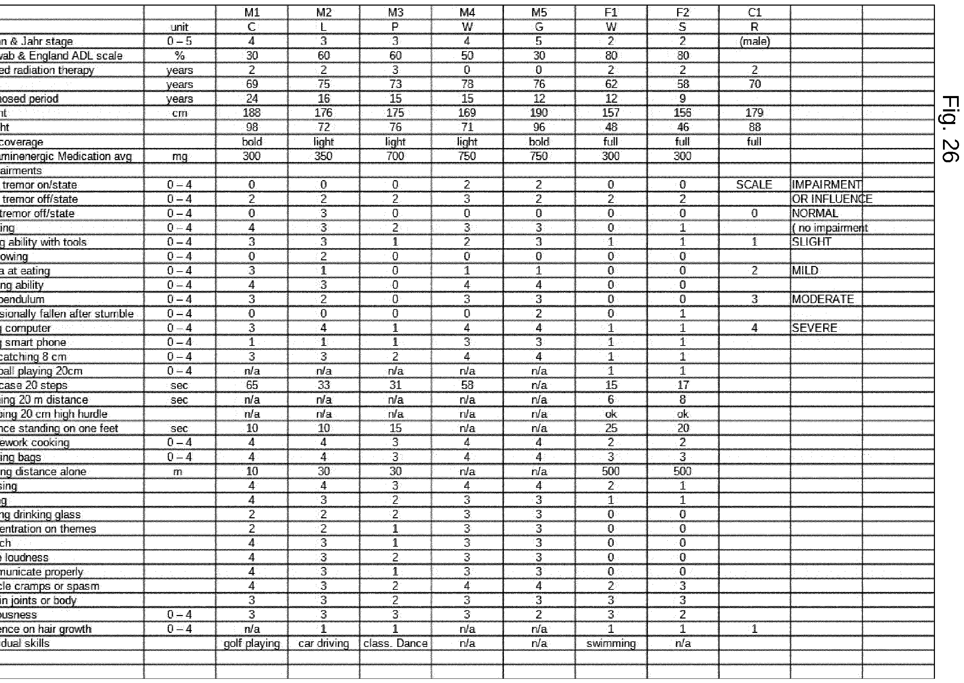

Fig 26. is a summary table of different assessments comparing the abilities and impairments of 7 patients and one control person.

### Method and outcome of study

Over a period of more than 2 years (November 2018 -June 2020) several systems of red /infra-red and blue light wavelength combinations were explored and tested on 7 patients ( 5 male, 2 female ), and 1 control person ( male ), ending up in an optimized quadruple-wavelength version of 660 nm 850 nm 1550 nm and 405 nm of irradiation LEDs, and therapy treatment time of 40 minutes twice a day
Safety was assessed on the basis of adverse events, regularly observation of behavior, cognition , mobility, dyskinesia, falls, adverse changes in mood, and impulsivity. Treatment Emergent Adverse Events ( TEAEs) were defined as adverse events, starting on or after the date of the first use of radiation device.

Derived from Unified Parkinson's Disease Rating Scale (UDPRS), Hoehn and Yahr Scale, Schwab and England Activities of Daily Living Scale (ADL), and Parkinson's Disease Questionnaire (PDQ-39) tests and observations were expanded with outdoor activities to get a broader view of patients impairments, abilities and disabilities.

Fig. 26 is comparing abilities and impairments of 7 patients and 1 control person.

As in this situation a randomized double-blinded clinical study is not feasible, the study is not fully based on statistical data, but subjective observation, discussions and interviews by the examiner, not all data have quantitative data sets, but major findings and observations at the group attending the radiation therapy, are :
1.) no progression of Parkinson's related disabilities.
2.) reduction of medication intake of more than 50%.
3.) stable movement abilities with minor tendencies to improvement of abilities.
4.) improved freshness, spirit and motivation
5.) radiation therapy is good tolerable, without treatment emergent adverse events. Whereas the group of patients treated with conventional dopaminergic medication was found to have:
6) dramatic ( within a period of one year) progressive degeneration of movement abilities, as other worsening factor, till to hospitalization and depending on walker rollator. Further:
7) no TEAEs at control group, but improved freshness and agility.

### Summary

The invention is comprising a portable-, wearable helmet-shaped head cover, as shown in Fig. 16 , containing an outer shell base-ABS part 96 , a removable outer-shell top-ABS part 95 , with a combined Light Emitting Diode ( LED ) array Fig. 3 emitting incoherent light at a wavelength of between 405 nm and 1550 nm, and an integrated LASER module Fig. 6 emitting coherent infra-red light at a wavelength of 850 nm, as illustrated in Fig. 14 a flexible printed circuit board ( PCB ) 90 , shaped according a spherical cutting pattern to achieve sufficient ventilation slots 92 , with an onboard Master Control Unit ( MCU ) 91 placed under the removable outer shell top ABS part, as well as sketched in Fig 3 includes 1440 Light Emitting Diodes ( LED ) as non-coherent light sources in bandwidth of 660 nm ±20 nm 40 , 850 nm ±20 nm 41, 1550 nm ±20 nm 42 and 405 nm ±20 nm 45 shown in Fig. 5, with drawing of side-view 43 and bottom-view 44 together with electrical chart of the quadruple light source 46 , mounted as single chip on a 5050 LED socket 47 thereafter soldered on a PCB as surface mounted devices (SMD) as shown in Fig. 4.

An optical grade transparent Polymethyl Methacrylate ( PMMA ) inner shell, Fig. 19 , protecting the emitting light sources, assuring irradiation without loss and hygienic clean ability for multiple usage.

The device is intended to support sub-and intra-cellular neuronal bio-chemical processes, leading in reduction of stiffness, reducing movement disorders, and promoting relaxation of muscles, whereas the VCSEL laser adapter at ear delivers normal radiation therapy through the skull, but used on nose, it is intended to breach the blood-brain-barrier( BBB ), allowing drugs delivery via the nasal cavity inside the brain, known as Intranasal Nose-to Brain Delivery. The full size helmet style, shown in Fig. 21, is covering forehead, ears, and back head till to neck, with a full irradiation influence on total skull 103 emitted by tight arranged light sources 102.

## Claims

1. A portable helmet-shaped device configured to cover patient's head, the device comprising
a PCB (39) and an array including 1440 non-coherent light sources grouped as quadruple light sources (46),
wherein each quadruple light source (46) has an arrangement of four LEDs comprising
a first LED (40, D1) configured to emit light in a bandwidth of 660 nm ±20 nm,
a second LED (41, D2) configured to emit light in a bandwidth of 850 nm ±20 nm,
a third LED (42, D3) configured to emit light in a bandwidth of 1550 nm ±20 nm and
a fourth LED (45, D4) configured to emit light in a bandwidth of 405 nm ±20 nm,
wherein each said arrangement of four LEDs (40, 41, 42, 45; D1, D2, D3, D4) is mounted on a single LED socket (38, 47) being soldered on said PCB (39) as SMD, and
wherein said array is configured to irradiate patient's head for stimulating bio-chemical effects to ease patient's impairments due to Parkinson's disease.

2. The device of claim 1, comprising an integrated ear/nose module (55) with two coherent laser-light sources configured to irradiate in a bandwidth of 850 nm ±1 nm, for simultaneous usage of head-irradiation, and ear- or nose-irradiation.

3. Device of claim 1, configured to independently and sequentially control irradiation segments comprising five frontal segments (61), seven side and back segments (62), and a center-top segment (63).

4. Device of claim 1, configured to adjust intensity in the range of 10 - 64 mW/cm² , and to adjust duration in the range of 4 - 40 min.

5. Device of claim 1,configured to provide incremental power increase at every irradiation cycle according to a stage generator signal.

6. Device of claim 1, configured to provide selectable radiation patterns.

7. Device of claim 1, configured to provide real time monitoring, controlling and data processing of essential electrical and environmental parameters.

8. Device of claim 1, comprising up to 16 cascadable stations, simultaneously controllable, configured to acquire illumination data are for data analysis and diagnostic purpose.

9. Device of claim 1, wherein said LED socket (38, 47) is a 5050 LED socket.

10. Device of claim 1, configured to be remotely controlled.

11. Device of claim 1, configured to provide irradiation adjustable overlay pulse frequency in the range of 10 Hz - 100 Hz.

## Patentansprüche

1. Eine tragbare helmförmige Vorrichtung, die so konfiguriert ist, dass sie den Kopf eines Patienten bedeckt, wobei die Vorrichtung eine Leiterplatte (PCB) (39) und ein Array umfasst, das 1440 nichtkohärente Lichtquellen enthält, die als vierfache Lichtquellen (46) gruppiert sind, wobei jede vierfache Lichtquelle (46) eine Anordnung von vier LEDs umfasst, bestehend aus einer ersten LED (40, D1), die so konfiguriert ist, dass sie Licht in einer Bandbreite von 660 nm ±20 nm aussendet, einer zweiten LED (41, D2), die so konfiguriert ist, dass sie Licht in einer Bandbreite von 850 nm ±20 nm aussendet, einer dritten LED (42, D3), die so konfiguriert ist, dass sie Licht in einer Bandbreite von 1550 nm ±20 nm aussendet, und einer vierten LED (45, D4), die so konfiguriert ist, dass sie Licht in einer Bandbreite von 405 nm ±20 nm aussendet, wobei jede genannte Anordnung von vier LEDs (40, 41, 42, 45; D1, D2, D3, D4) auf einem einzigen LED-Sockel (38, 47) montiert und als SMD auf die genannte Leiterplatte (PCB) (39) gelötet ist, und wobei das genannte Array so konfiguriert ist, dass es den Kopf des Patienten bestrahlt, um biochemische Effekte zu stimulieren, die Beeinträchtigungen des Patienten aufgrund der Parkinson-Krankheit lindern.

2. Die Vorrichtung nach Anspruch 1, umfassend ein integriertes Ohr-/Nasensystem (55) mit zwei kohärenten Laserlichtquellen, die so konfiguriert sind, dass sie in einer Bandbreite von 850 nm ±1 nm bestrahlen, zur gleichzeitigen Nutzung der Kopfbestrahlung sowie der Ohr- oder Nasenbestrahlung.

3. Die Vorrichtung nach Anspruch 1, die so konfiguriert ist, dass sie die Bestrahlungssegmente unabhängig und nacheinander steuert, wobei fünf vordere Segmente (61), sieben Seiten- und Rückensegmente (62) und ein zentrales Obersegment (63) enthalten sind.

4. Die Vorrichtung nach Anspruch 1, die so konfiguriert ist, dass sie die Intensität im Bereich von 10 - 64 mW/cm² und die Dauer im Bereich von 4 - 40 Minuten einstellt.

5. Die Vorrichtung nach Anspruch 1, die so konfiguriert ist, dass sie bei jedem Bestrahlungszyklus eine inkrementelle Leistungssteigerung gemäß einem Stufengeneratorsignal bereitstellt.

6. Die Vorrichtung nach Anspruch 1, die so konfiguriert ist, dass sie auswählbare Strahlungsmuster bereitstellt.

7. Die Vorrichtung nach Anspruch 1, die so konfiguriert ist, dass sie die Überwachung, Steuerung und Datenverarbeitung wesentlicher elektrischer und umweltbedingter Parameter in Echtzeit ermöglicht.

8. Die Vorrichtung nach Anspruch 1, umfassend bis zu 16 kaskadierbare Stationen, die gleichzeitig steuerbar sind und so konfiguriert sind, dass sie Beleuchtungsdaten zu Analyse- und Diagnosezwecken erfassen.

9. Die Vorrichtung nach Anspruch 1, wobei der genannte LED-Sockel (38, 47) ein 5050-LED-Sockel ist.

10. Die Vorrichtung nach Anspruch 1, die so konfiguriert ist, dass sie fernsteuerbar ist.

11. Die Vorrichtung nach Anspruch 1, die so konfiguriert ist, dass sie eine einstellbare Pulsfrequenzüberlagerung im Bereich von 10 Hz bis 100 Hz zur Bestrahlung bereitstellt.

## Revendications

1. Un dispositif portable en forme de casque, configuré pour couvrir la tête d'un patient, le dispositif comprenant un circuit imprimé (PCB) (39) et un réseau comprenant 1440 sources lumineuses non cohérentes groupées en sources lumineuses quadruples (46), chaque source lumineuse quadruple (46) ayant une disposition de quatre LED comprenant une première LED (40, D1) configurée pour émettre de la lumière dans une bande passante de 660 nm ±20 nm, une deuxième LED (41, D2) configurée pour émettre de la lumière dans une bande passante de 850 nm ±20 nm, une troisième LED (42, D3) configurée pour émettre de la lumière dans une bande passante de 1550 nm ±20 nm et une quatrième LED (45, D4) configurée pour émettre de la lumière dans une bande passante de 405 nm ±20 nm, chaque disposition des quatre LED (40, 41, 42, 45; D1, D2, D3, D4) étant montée sur une seule prise LED (38, 47) soudée sur ledit PCB (39) en tant que SMD, et ledit réseau étant configuré pour irradier la tête du patient afin de stimuler des effets biochimiques pour atténuer les déficiences du patient dues à la maladie de Parkinson.

2. Le dispositif selon la revendication 1, comprenant un module intégré oreille/nez (55) avec deux sources lumineuses laser cohérentes configurées pour irradier dans une bande passante de 850 nm ±1 nm, pour une utilisation simultanée de l'irradiation de la tête, et de l'irradiation de l'oreille ou du nez.

3. Dispositif selon la revendication 1, configuré pour contrôler indépendamment et séquentiellement des segments d'irradiation comprenant cinq segments frontaux (61), sept segments latéraux et arrière (62), et un segment central supérieur (63).

4. Dispositif selon la revendication 1, configuré pour ajuster l'intensité dans une plage de 10 à 64 mW/cm², et ajuster la durée dans une plage de 4 à 40 minutes.

5. Dispositif selon la revendication 1, configuré pour fournir une augmentation progressive de la puissance à chaque cycle d'irradiation selon un signal de générateur d'étapes.

6. Dispositif selon la revendication 1, configuré pour fournir des motifs de radiation sélectionnables.

7. Dispositif selon la revendication 1, configuré pour fournir une surveillance en temps réel, un contrôle et un traitement des données des paramètres électriques et environnementaux essentiels.

8. Dispositif selon la revendication 1, comprenant jusqu'à 16 stations en cascade, contrôlables simultanément, configurées pour acquérir des données d'illumination à des fins d'analyse et de diagnostic.

9. Dispositif selon la revendication 1, où ladite prise LED (38, 47) est une prise LED 5050.

10. Dispositif selon la revendication 1, configuré pour être contrôlé à distance.

11. Dispositif selon la revendication 1, configuré pour fournir une fréquence de pulsation superposée ajustable dans une plage de 10 Hz à 100 Hz.
